(19) Europäisches Patentamt
European Patent Office
Office européen des brevets

(11) **EP 0 547 025 B2**

(12) **NEW EUROPEAN PATENT SPECIFICATION**

(45) Date of publication and mention
of the opposition decision:
**19.06.2002 Bulletin 2002/25**

(45) Mention of the grant of the patent:
**12.06.1996 Bulletin 1996/24**

(21) Application number: **93100634.0**

(22) Date of filing: **11.02.1989**

(51) Int Cl.7: **A61M 1/14**, A61M 1/34

(54) **Method for determining a concentration of a substance in blood or the dialysance of a dialyser**

Verfahren zur Bestimmung der Konzentration einer Substanz in Blut oder der dialysanz eines Dialysators

Méthode pour déterminer la concentration d'une substance dans le sang ou la dialysance d'un dialyseur.

(84) Designated Contracting States:
**BE CH DE ES FR GB IT LI NL**

(30) Priority: **03.03.1988 SE 8800757**

(43) Date of publication of application:
**16.06.1993 Bulletin 1993/24**

(62) Document number(s) of the earlier application(s) in accordance with Art. 76 EPC:
**89102407.7 / 0 330 892**

(73) Proprietor: **GAMBRO AB**
**220 10 Lund (SE)**

(72) Inventor: **Sternby, Jan Peter**
**S-226 52 Lund (SE)**

(74) Representative: **Lejeune, Daniel**
**Gambro Patent Department**
**61, avenue Tony Garnier**
**69007 Lyon (FR)**

(56) References cited:
EP-A- 0 097 366    EP-B- 0 428 927
WO-A-86/00239    DE-A- 2 825 134
DE-C- 3 938 662    US-A- 5 100 554

• SARGENT, J.A. , GOTCH, F.A. " Principles and Biophysics of Dialysis " in: " REPLACEMENT OF RENAL FUNCTION BY DIALYSIS " , page 53 - 96
• W.DRUKKER, F.M. PARSONS, J.F. MAHLER (EDITORS), NIJHOFF PUBLISHER, DEN HAAG, 1983
• 1983
• H. E. Franz: "Blutreinigungsverfahren", Georg Thieme Verlag Stuttgart, New York, 1990, S. 479-492
• Instructions for use NEPHRAL, Hospal, 1996

EP 0 547 025 B2

## Description

TECHNICAL FIELD

The present invention relates to a method for determining a concentration of a substance in the blood of a patient undergoing a dialysis treatment in an artificial kidney and/or the actual dialysance for said substance of the artificial kidney, the artificial kidney comprising an extracorporeal blood circuit connected to a dialyser having a semipermeable membrane de-limiting a first compartment for the circulation of blood on one side of the membrane and a second compartment for circulating a dialysis liquid on the other side of the membrane.

The term dialysis treatment here also comprises similar treatments, e.g haemodiafiltration, that is dialysis with high ultrafiltration and addition of a replacement liquid.

BACKGROUND ART

In a normal dialysis treatment there is normally no feedback of patient's data to the dialysis machine. Thus the patient's temperature, blood values etc. may be no matter how abnormal without the machine reacting, as long as the machine's values are correct

As an example of systems which control the composition of the dialysis liquid irrespectively of the blood parameters of the individual patient can be mentioned, for example, those described in the US patents 4 158 034, 4 293 409 and 4 508 622.

The present invention may be said also to constitute a further development of the dialysis system developed by the Gambro Group, which is marketed under the name of Gambro AK-10 and Gambro AK-100. Details of such systems are described more fully, for example, in the two first mentioned US patents and in the US patents 4 194 974, 4 191 359, 4 585 552 and 4 536 201 and the European patent publications EP-B-22 922, EP-A-204 260, EP-A-204 174, EP-A-208 090 and EP-A-238 809.

DISCLOSURE OF INVENTION

It is an object of the present invention to increase the patient's comfort by making the treatment more individualized. This can be done in that certain parameters of the patient are measured and adapted to control the dialysis machine used. Examples of such parameters may be blood pressure, temperature, blood gas content, electrolyte content, pH-status etc. Such measurements should be made wholly without blood contact, so that transmitters etc. need not be of the disposable type or be sterilized between each treatment.

The method in accordance with the invention is characterized by the steps of:

- circulating successively in the second compartment of the dialyser a first and a second dialysis liquid having different concentrations of the substance, the first and the second liquid differing only by the concentration of the substance,
- measuring in the first and second dialysis liquids the conductivity or the concentration of the substance upstream and downstream of the dialyser, and
- calculating from the measured conductivity or concentration of the substance in the first and second dialysis liquids, the concentration of the substance in the blood at the inlet of the dialyser and/or the actual dialysance of the artificial kidney.

The method can be used simply for monitoring the composition of the dialysis liquid and thereby an indirect passive monitoring of the composition of the blood. Preferably, the method in accordance with the invention uses the measurements for the calculation of a blood parameter which is altered as a function of the parameter measured, for the purpose of calculation and/or control of this blood parameter in the patient.

Inasfar as the electrolyte composition of the blood is concerned, it has been found that it is a prerequisite to obtain a value of this by measuring the conductivity of the dialysis liquid which passes the dialyser. Through the influence of the blood the conductivity of the incoming dialysis liquid will be altered and the magnitude of the alteration will depend on the composition of the blood. Since the conductivity for the most part is produced by sodium ions together with its anions, the conductivity largely becomes a measure of the sodium content in the dialysis liquid and in the blood. Preferably, therefore, the method in accordance with the invention measures the conductivity before and after the dialyser for the purpose of calculation and/or control of the conductivity and/or the sodium content of the blood.

Alternatively, the dialysis method in accordance with the invention can be provided with one or more ion-selective electrodes for measuring the content of one or more defined ions in the dialysis liquid for the purpose of calculation and/or control of the corresponding ion content in the blood. In this way other ions beside sodium can also be measured, such as for example calcium and potassium.

It will be obvious to those versed in the art, that many other blood parameters, too, can be calculated and/or controlled with the help of corresponding values measured on the dialysis liquid side. Thus, it should be possible, for example, to use in accordance with the invention, a gas analyser arranged after the dialyser for measuring the gas content in the dialysis liquid for the purpose of calculation and/or control of corresponding blood gases. However, this has not yet been verified in practice.

It will be obvious, moreover, to those versed in the art that it would be better to measure directly various ions with the help of ion-selective transmitters instead of taking the route via a conductivity meter. With the technique available today, however, a conductivity measurement is appreciably more stable, more accurate, simpler and less expensive. Preferably, therefore, conductivity measurement is used. It is intended then with the help of this to measure the conductivity on the diaysis liquid side so as to calculate thereafter theoretically the conductivity on the blood side. On the assumption that the conductivity mainly depends upon the sodium content, the sodium passage or sodium dialysance will be determining for the effect of the blood side on the conductivity value of the dia-lysis liquid measured after the dialyser. A simple mass balance for sodium at constant flow and neglecting the so-called Donnan effect, gives the formula:

$$Cdout = Cdin + (Cbin - Cdin) \times K$$

wherein
Cdout = the conductivity or the concentration of sodium in used dialysis liquid;
Cdin = the conductivity or the concentration of sodium in fresh dialysis liquid;
Cbin = the conductivity or the concentration of sodium in untreated blood;
K = proportionality constant depending on the flow.
If the ultrafiltration is kept at zero, K becomes D/Qd (=relative dialysance) where
D = dialysance value for sodium/conductivity;
Qd = dialysis liquid flow.
The same formula applies also where the ultrafiltration is different from zero, but with a modified value of K.

The factor K can be determined in two different ways. The simplest is, starting out from a knowledge of dialyser, blood flow and liquid flow, to determine it empirically. It is also possible to determine K continuosly by a regular change of the set conductivity of the liquid. At each change a measure of K is obtained from a comparison between the conductivity measured before and after the dialyser, respectively. This method requires a liquid conductivity which during the treatment is altered by steps, e.g. every 6th minute by 0.5 mS/cm, up or down respectively from the set value which ought to be in the order of magnitude 13.7 - 14.0 mS/cm. These changes are so rapid, that the body quite likely would not keep up with them to any appreciable extent. At the same time they are so small that they do not notably affect the afore-mentioned formula.

The dialysis method in accordance with the invention appropriately is provided with means for the control of the composition of the dialysis liquid before the dialyser as a function of the parameter measured in such a manner that the dialysis result becomes a direct function of the corresponding blood parameter. As a result, conditions on the blood side will be directly determining for the dialysis and not the preset values on the dialysis liquid side, as has been the case normally up to now. If the dialysis method in accordance with the invention is adapted for determination of the conductivity and/or the sodium content in the blood, the said means for adjusting of the composition of the dialysis liquid before the dialyser may be adapted to bring about an equilibrium between the conductivities in the dialysis liquid and in the blood. In this manner a conductivity adapted to the individual is obtained, which should provide maximum comfort for the patient.

The dialysis method in accordance with the invention comprises, in a manner known per se, means for measuring the actual dialsyis liquid parameter, which is measured after the dialyser and also just before the same. The system suitably is provided also with a shunt line for conducting the fresh dialysis liquid past the dialyser directly to the measuring device normally placed after the dialyser, for the purpose of calibration of the measuring devices used. It should be noted in this connection that any minor fault common to both measuring devices is of less importance for the final result than if only one of the measuring devices were to present an incorrect measuring value.

The dialysis method in accordance with the invention, in a manner know per se, comprises means for the measuring of the said parameter of the dialysis liquid both before and after the dialyser. In such case the parameter measured after the dialyser instead may be compared with the value of the same parameter measured before the dialyser. In practice it has been found simpler, however, to use a corresponding set value for comparison. This set value, of course, has already been entered into the system.

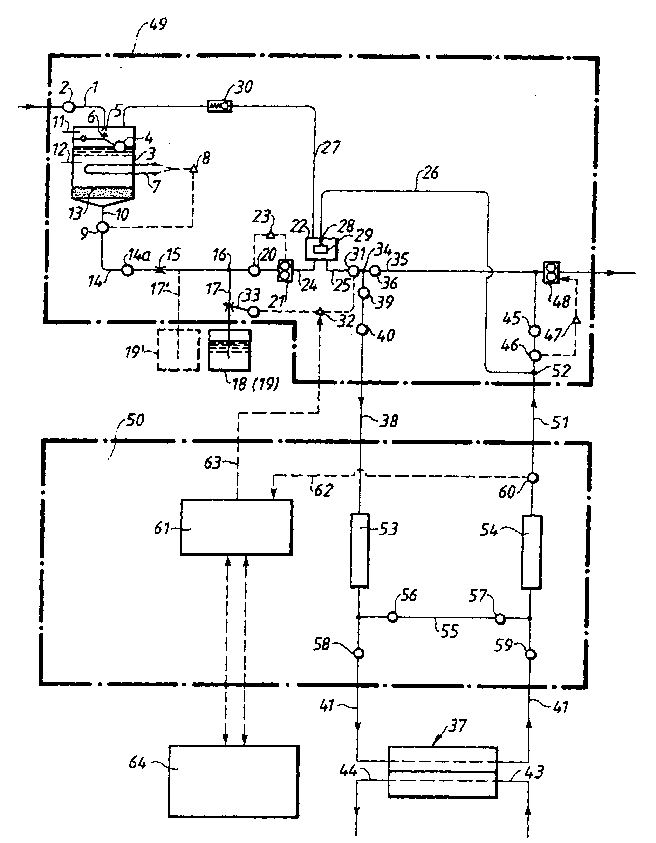

BRIEF DESCRIPTION OF THE DRAWING

The invention is described in more detail below with reference to the attached drawing which is a block diagram comprising a mixing module, a flow control module, an external computer and a dialyser.

BEST MODE OF CARRYING OUT THE INVENTION

On the drawing an inlet duct for fresh water is designated 1. Thus duct 1, which is provided with an inlet valve 2, leads to a water vessel 3. This vessel 3 is provided with a float valve 4 which is adapted to close the water intake with the help of a closing cone 6 when the water vessel has been filled. The water vessel, moreover, comprises means 7 for heatting the water, e. g. in the form of a heating loop. This heating loop is controlled by a temperature regulating means 8 which in turn is controlled by a temperature transmitter 9 in the outlet duct 10 from the vessel 3. Furthermore, the vessel 3 comprises maximum and minimum monitors 11 and 12, respectively, shown schematically, for sensing the liquid level in the vessel. These level monitors may be adapted, for example, to control directly the inlet valve 2. Finally the vessel contains a filter element 13, shown schematically, which in the first place is intended to remove any solid particles from the water, but which in practice also removes a certain amount of gas, e.g. free gas bubbles.

After heating the fresh water supplied is conducted via the outlet line 10, the temperature transmitter 9, a duct 14 with a shut-off valve 14a, and a throttle valve 15 to a branching point 16. Here a branch 17 is connected which normally starts out from a source 18 of salt solution concentrate. Generally this source quite simply consists of a drum with a salt solution. When the system shown is to be sterilized, however, this drum is replaced by one containing a sterilizing agent which is marked (19) in the figure. In practice this is the simplest solution. It would also be possible of course, would this be required for any special reason, to connect a further line 17 to the system parallel with the line 17 for the connection of a separate vessel 19'. This has been indicated in the figure by broken lines.

From the point 16 the liquidflows via pressure gauge 20 and a pump 21 to a bubble separator 22. The pressure gauge 20 here controls the pump via a pressure regulating means 23. The supply line to the bubble separator 22 has been designated 24.

From the bubble separator 22 starts out beside the ordinary line 25, also a line 26 for the removal of the separated gas, and also a return line 27. The inlet 28 to the line 26 is controlled by a float valve 29 which closes this inlet when the bubble separator 22 is filled with liquid in connection with sterilization. The return line 27 is provided with a spring-loaded check valve 30 and leads back to the liquid vessel 3.

The line 25 leads to a conductivity meter 31 which via a control means 32 controls a throttle valve 33 in the line 17 for salt solution concentrate. Alternatively it may control instead a concentrate pump in the same line 17. After the conductivity meter 31 the liquid flow reaches a new branching point 34 from which originates shunt line 35 with a valve 36. This shunt line 35 is used when it is desired to couple the liquid flow past the dialyser, e g when an abnormality is detected in the dialysis liquid, for example, of its temperature or salt content. Normally the liquid otherwise flows to the dialyser 37 via a line 38 which contains a valve 39 and a flow meter 40. On its way to the dialyse 37 the dialysis liquid passes a flow control module which, as a whole, is designated 50. The components described up to now, on the other hand, are included in a liquid preparation module which, as a whole, is designated 49. On its way from the dialyser 37 the dialysis liquid once agains passes the flow control module 50 to be returned via a line 51 to the module 49. At a point 52 the line 51 is coupled together with the line 26 from the bubble separator 22.

The lines or tubings which are normally coupled to the dialyser 37 are designated 41. During sterilization, though, these tubings are coupled to a "safety-bypass", not shown in the drawing. An example of such a "safety-bypass" is described in the US patent 4 122 010 and it need not be described in greater detail, therefore, in connection with the present invention. The inlet and outlet respectively on the blood side of the dialyser are designated 43 and 44.

A blood detector 45 provided in the module 49 raises an alarm which possibly shuts down the whole system if blood is detected in the dialysis liquid. This blood detector, for example, may be a transparent tubing opposite an otherwise shielded photocell device which directly senses the occurrence of any blood in the dialysis liquid. Before the blood detector a pressure gauge 46 is passed which via a control means 47 controls a liquid pump 48. Thereafter the liquid flow is conducted finally to a drain, not shown.

In the module 50 the dialysis liquid flow to the dialyser is measured in a first measuring device 53. In the same manner the dialysis liquid flow from the dialyser is measured in a second measuring device 54. Through a comparison of the values obtained the ultrafiltration in the dialyser can be determined. The two measuring devices 53 and 54 may be included, for example, in a differential measuring arrangement of the type which is described in British patent 2 003 274 or in the US patent 4 585 552. During the calibration of the measuring devices the dialyser is shunted with the help of a bypass line 55 comprising two valves 56 and 57. At the same time the lines to and from the dialyser are shut off with the help of valves 58 and 59 respectively.

After the dialysis liquid flow has passed the measuring device 54 it passes a device 60 for the measurement of at least one parameter representative for it. In the example shown this device consists of a conductivity meter. The value measured is passed to a microprocessor 61, which is indicated by the broken line 62. Here the measured value is compared with the set value of the same parameter before the dialyser, that is to say in this case the conductivity. This set value is adjusted with the help of the microprocessor 61 which is indicated by the broken line 63. This takes place thus with the help of the afore-mentioned control means 32. If desired the microprocessor 61 may be provided furthermore with data from or it may furnish data to a further personal computer 64. With the help of this further computer, for example, the individual values of the patient can be transferred to the microprocessor 61 at the same time as the values calculated for the blood side of the dialyser can be noted.

Naturally the invention is not limited solely to the embodiment described above but can be varied within the frame-work of the subsequent claims. For example, the system in accordance with the example of an application described above is based on only one concentrate, that is to say that taken from the container 18. The invention also may be applied, however, e.g. when two concentrates are used in the manner which is described in EP-B1-22922. The value measured in the device 60 is compared in such a case with the ready-mixed dialysis liquid, that is to say after mixing in of the two concentrates used. The two concentrates, for the rest, need not be in liquid form. Concentrate in powder form may also be used e.g. in accordance with what is described in European patent EP-B-278 100.

## Claims

1. A method for determining a concentration of a substance in the blood of a patient undergoing a dialysis treatment in an artificial kidney and/or the actual dialysance for said substance of the artificial kidney, the artificial kidney comprising an extracorporeal blood circuit connected to a dialyzer having a semipermeable membrane delimiting a first compartment for the circulation of blood on one side of the membrane and a second compartment for circulating a dialysis liquid on the other side of the membrane, **characterized by** the steps of:

   - circulating successively in the second compartment of the dialyser a first and a second dialysis liquid having different concentrations of the substance, the first and second liquid differing only by the concentration of the substance
   - measuring in the first and second dialysis liquids the conductivity or the concentration of the substance upstream and downstream of the dialyser, and
   - calculating from the measured conductivity or concentration of the substance in the first and second dialysis liquids, the concentration of the substance in the blood at the inlet of the dialyser and/or the actual dialysance of the artificial kidney.

2. A method according to claim 1, **characterized in that** the concentration of the substance in blood and/or the actual dialysance of the artificial kidney is calculated according to the formula:

$$Cd\ out = Cd\ in + (Cb\ in - Cd\ in) \times D/Qd$$

   wherein
   Cd in = conductivity of the dialysis liquid or concentration of the substance in the dialysis liquid upstream of the dialyser;
   Cd out = conductivity of the dialysis liquid or concentration of the substance in the dialysis liquid downstream of the dialyser;
   Cb in = concentration of the substance in the blood upstream of the dialyser;
   D = dialysance of the artificial kidney for the substance;
   Qd = flow rate of the dialysis liquid.

3. A method according to claim 1 or 2, **characterized** **in that** the measuring of the conductivity or the concentration of the substance upstream of the dialyser is replaced by a set value of the conductivity or concentration 4

4. A method according to claim 1, 2 or 3, **characterized in that** the substance is sodium and **in that** the conductivity of the dialysis liquid is measured upstream and downstream of the dialyser.

## Patentansprüche

1. Verfahren zum Bestimmen einer Konzentration einer Substanz im Blut eines Patienten, der sich einer Dialysebehandlung in einer künstlichen Niere unterzieht, und/oder der tatsächlichen Dialysierfähigkeit der künstlichen Niere für die Substanz, wobei die künstliche Niere einen extrakorporalen Blutkreislauf aufweist, der an eine Dialyseeinrichtung angeschlossen ist mit einer semipermeablen Membran, welche ein erstes Abteil für die Zirkulation von Blut auf einer Seite der Membran und ein zweites Abteil für die Zirkulation einer Dialyseflüssigkeit auf der anderen Seite der Membran abgrenzt, **gekennzeichnet durch** die Schritte :

   - Sukzessives Zirkulieren einer ersten und einer zweiten Dialyseflüssigkeit in dem zweiten Abteil der Dialyseeinrichtung, wobei die Flüssigkeiten unterschiedliche Konzentrationen der Substanz haben, und wobei die erste Flüssigkeit von der zweite nur **durch** die Konzentration der Substanz sich unterscheidet,
   - Messen der Leitfähigkeit oder der Konzentration der Substanz aufstromig und abstromig von der Dialyseeinrichtung in der ersten und der zweiten Dialyseflüssigkeit und
   - Berechnen der Konzentration der Substanz im Blut am Einlaß der Dialyseeinrichtung und/oder der tatsächlichen Dialysierfähigkeit der künstlichen Niere aus der gemessenen Leitfähigkeit oder Konzentration der Substanz in der ersten und der zweiten Dialyseflüssigkeit.

2. Verfahren nach Anspruch 1, **dadurch gekennzeichnet, daß** die Konzentration der Substanz im Blut und/oder die tatsächliche Dialysierfähigkeit der künstlichen Niere nach der Formel berechnet wird :

$$Cd\ out = Cd\ in + (Cb\ in - Cd\ in) \times D/Qd$$

   wobei

   - Cd in = Leitfähigkeit der Dialyseflüssigkeit oder Konzentration der Substanz in der Dialyseflüssigkeit aufstromig von der Dialyseeinrichtung ;

- Cd out = Leitfähigkeit der Dialyseflüssigkeit oder Konzentration der Substanz in der Dialyseflüssigkeit abstromig von der Dialyseeinrichtung ;
- Cb in = Konzentration der Substanz in dem Blut aufstromig der Dialyseeinrichtung ;
- D = Dialysierfähigkeit der künstlichen Niere für die Substanz ;
- Qd = Flußgeschwindigkeit der Dialysierflüssigkeit.

3. Verfahren nach Anspruch 1 oder 2, **dadurch gekennzeichnet, daß** das Messen der Leitfähigkeit oder der Konzentration der Substanz aufstromig von der Dialysiereinrichtung durch einen Sollwert der Leitfähigkeit oder Konzentration ersetzt wird.

4. Verfahren nach Anspruch 1, 2 oder 3, **dadurch gekennzeichnet, daß** die Substanz Natrium ist und daß die Leitfähigkeit der Dialyseflüssigkeit aufstromig und abstromig von der Dialyseeinrichtung gemessen wird.

## Revendications

1. Méthode pour déterminer une concentration d'une substance dans le sang d'un patient subissant un traitement de dialyse dans un rein artificiel et/ou la dialysance effective pour la dite substance du rein artificiel, le rein artificiel comprenant u n circuit de sang extracorporel connecté à un dialyseur ayant une membrane semiperméable qui délimite un premier compartiment pour la circulation de sang sur une face de la membrane et un deuxième compartiment pour la circulation d'un liquide de dialyse sur l'autre face de la membrane, **caractérisée par** les étapes de :

- mise en circulation successivement dans le deuxième compartiment du dialyseur, d'un premier et d'un deuxième liquides de dialyse ayant des concentrations différentes de la substance, le premier et le second liquide différant seulement par la concentration de la substance,
- mesure, dans les premier et deuxième liquides de dialyse, de la conductivité ou de la concentration de la substance en amont et en aval du dialyseur, et
- calcul, à partir de la conductivité ou de la concentration mesurée de la substance dans les premier et deuxième liquides de dialyse, de la concentration de la substance dans le sang à l'entrée du dialyseur et/ou de la dialysance effective du rein artificiel.

2. Méthode suivant la revendication 1, **caractérisée en ce que** la concentration de la substance dans le sang et/ou la dialysance effective du rein artificiel est calculée conformément à la formule :

$$Cd\ out = Cd\ in + (Cb\ in - Cd\ in) \times D/Qd$$

dans laquelle

- Cd in = conductivité du liquide de dialyse ou concentration de la substance dans le liquide de dialyse en amont du dialyseur ;
- Cd out = conductivité du liquide de dialyse ou concentration de la substance dans le liquide de dialyse en aval du dialyseur;
- Cb in = concentration de la substance dans le sang en amont du dialyseur ;
- D = dialysance du rein artificiel pour la substance ;
- Qd = débit du liquide de dialyse.

3. Méthode suivant la revendication 1 ou 2, **caractérisée en ce que** la mesure de la conductivité ou de la concentration de la substance en amont du dialyseur est remplacée par une valeur de consigne de la conductivité ou de la concentration.

4. Méthode suivant la revendication 1, 2 ou 3, **caractérisée en ce que** la substance est le sodium et **en ce que** la conductivité du liquide de dialyse est mesurée en amont et en aval du dialyseur.